# EUROPÄISCHE PATENTANMELDUNG

(11) **EP 1 559 380 A2**
(43) Veröffentlichungstag der Anmeldung: **03.08.2005**
(21) Anmeldenummer: 05001309.3
(22) Anmeldetag: 24.01.2005
(51) Int. Cl.: A61C 1/00

(54) **Verfahren und Vorrichtung zur Bestimmung des zum Aufbringen eines Drehmoments benötigten Werts des Motorstroms für medizinische Handstücke**

(30) Priorität: 29.01.2004 AT 1182004
(71) Anmelder: W & H Dentalwerk Bürmoos GmbH, 5111 Bürmoos (AT)
(72) Erfinder: Mangelberger, Michael, 5113 St.Georgen bei Salzburg (AT); Schröck, Rainer, 5112 Lamprechtshausen (AT)

(57) **Zusammenfassung**

Die vorliegende Erfindung betrifft ein Verfahren und eine Vorrichtung (1) zur Bestimmung des zum Aufbringen eines Drehmoments benötigten Werts des Motorstroms für medizinische Handstücke (2) sowie eine Steuervorrichtung für medizinische Handstücke (2) mit einer Vorrichtung (1) zur Bestimmung des zum Aufbringen eines Drehmoments benötigten Werts des Motorstroms für medizinische Handstücke (2). Um zum Anwählen und Starten des Verfahrens das Berühren des Bedienfelds bzw. der Tasten der Steuervorrichtung unnötig zu machen, wird die Vorrichtung (1) zur Bestimmung des Motorstroms mit einem Element zur Signalabgabe (13, 20, 30, 40, 60), bevorzugt ein Sensor oder ein Schaltelement, ausgestattet, das ein Signal zum Start des Verfahrens abgibt, sobald die Verbindung zwischen der Drehmomentbremse (9) und der Werkzeughalterung des Handstücks (2) hergestellt ist.

## Beschreibung

Die vorliegende Erfindung betrifft ein Verfahren und eine Vorrichtung zur Bestimmung des zum Aufbringen eines Drehmoments benötigten Werts des Motorstroms für medizinische Handstücke sowie eine Steuervorrichtung mit einer Vorrichtung zur Bestimmung des zum Aufbringen eines Drehmoments benötigten Werts des Motorstroms für medizinische Handstücke.

Eine derartige Vorrichtung ist aus der EP 1 084 683 A2 (US 6,607,385 B1) bekannt, in der auch der medizinische Hintergrund für die Verwendung sowie die durch die Verwendung der Vorrichtung resultierenden Vorteile beschrieben sind.
Die Anmelderin vertreibt seit einiger Zeit eine derartige Vorrichtung als Teil einer Steuervorrichtung eines Elektromotors mit dem medizinische Handstücke angetrieben werden. Wünscht der Anwender den Wert des Motorstroms zum Aufbringen des Drehmoments für sein Handstück zu bestimmen bzw. zu begrenzen, so führt er das Verfahren (im folgenden auch als Kalibrierverfahren bezeichnet) gemäß EP 1 084 683 A2 (US 6,607,385 B1) durch, wobei er zuerst das erste Ende eines Verbindungsstifts in die Werkzeughalterung des Handstücks und anschließend das zweite Ende des Stifts in die Aufnahme der Vorrichtung steckt, anschließend über Tasten eines Bedienfelds an der Vorderseite der Steuervorrichtung die Programmebene, in der das Kalibrierverfahren hinterlegt ist, anwählt und das Verfahren startet. Das Kalibrierverfahren selbst läuft anschließend vollkommen automatisiert gemäß einem im Speicher der Steuervorrichtung gespeicherten und vom Mikroprozessor der Steuervorrichtung verarbeiteten Programm ab.

Nachteilig bei diesem Verfahren ist für den Anwender die Auswahl und der Start des Kalibrierverfahrens mittels Tasten des Bedienfelds. Dies wird als umständlich und zeitraubend empfunden und es nötigt den Anwender, insbesondere wenn er während einer laufenden sterilen Behandlung das Kalibrierverfahren durchführen oder wiederholen möchte, das unsterile Bedienfeld bzw. die unsterilen Tasten zu berühren.

Der vorliegenden Erfindung liegt daher die Aufgabe zugrunde eine Vorrichtung zu schaffen bzw. ein Verfahren zu entwickeln, bei der / dem zum Anwählen und Starten des Kalibrierverfahrens das Berühren des unsterilen Bedienfelds bzw. der unsterilen Tasten nicht notwendig ist.

Diese Aufgabe wird gemäß der vorliegenden Erfindung durch eine Vorrichtung mit den Merkmalen des Anspruchs 1 und 12 und durch ein Verfahren nach Anspruch 13 gelöst.

Die erfindungsgemäße Vorrichtung zur Bestimmung des Motorstroms für medizinische Handstücke mit einer Drehmomentbremse, die mit der Werkzeughalterung des Handstücks verbindbar ist, einer Motorstrom-Meßvorrichtung für den Handstückmotor und, falls erforderlich, einer Drehmoment-Meßvorrichtung wird zusätzlich mit einem Element zur Signalabgabe ausgestattet, das ein Signal zum Start des Kalibrierverfahrens abgibt.

In einem ersten Ausführungsbeispiel ist das Element zur Signalabgabe als Sensor ausgebildet. Das abgegebene Signal wird von einer Auswerte-Elektronik empfangen und verarbeitet. Bevorzugt ist das Kalibrierverfahren als Programm in einem Speicher abgelegt, wobei ein Mikroprozessor, der auch die Auswerte-Elektronik umfaßt, nach Erhalt des Signals des Sensors das Programm aufruft und verarbeitet, d.h. die einzelnen Schritte des Kalibrierverfahrens laufen vollkommen automatisiert ab.

Bevorzugt erfolgt die Verbindung zwischen der Drehmomentbremse und der Werkzeughalterung des Handstücks mittels eines Verbindungsstifts, wobei die Drehmomentbremse mit einer Aufnahme (Bohrung) für den Verbindungsstift versehen ist und der Sensor oder Teile des Sensors an die Aufnahme angrenzen oder in die Aufnahme ragen. Die Signalabgabe des Sensors wird durch die Veränderung von Werten eines oder mehrerer Parameter ausgelöst. Bevorzugte Parameter können sein:
- Druck/Kraft - der als Drucksensor (zum Beispiel in Form eines Piezoelements oder Dehnmeßstreifens) ausgebildete Sensor detektiert den durch den Anwender während des Einsteckens oder nach dem Einstecken des Verbindungsstifts in die Aufnahme der Drehmomentbremse ausgeübten Druck.
- Rotation / Drehzahl - der als Drehzahlsensor (zum Beispiel in Form eines Hall-Sensors in Kombination mit einem Magneten oder einer Photozelle) ausgebildete Sensor detektiert die Rotation des Verbindungsstifts oder der Aufnahme, die durch Betätigung des Antriebs (Motors) des Handstücks in Rotation versetzt werden. Bevorzugt wird der Antrieb über eine Fuß- oder Fingersteuerung oder über eine Fernbedienung, die mit der Steuervorrichtung oder direkt mit dem Antrieb in Verbindung steht, betätigt.
- Drehmoment - der als Drehmomentsensor (zum Beispiel in Form einer Drehfederwaage oder eines Dehnmeßstreifens mit flexiblem Rotor) ausgebildete Sensor detektiert das vom Antrieb auf den Verbindungsstift übertragene Drehmoment.
- Lichtstärke - der Sensor (zum Beispiel in Form einer Photozelle) detektiert die Lichtstärke einer Strahlung einer Lichtquelle. Der Lichtstrahl verläuft durch die Aufnahme und wird durch das Einsetzen des Verbindungsstifts in die Aufnahme abgeschwächt oder unterbrochen.
- Lautstärke - der Sensor (zum Beispiel in Form eines Mikrofons) detektiert die Lautstärke eines akustischen Signals, das durch den Anwender, bevorzugt seine Stimme, abgegeben oder durch das Einsetzen des Verbindungsstifts in die Aufnahme, hervorgerufen wird.
- Magnetfeldstärke ― der Sensor (zum Beispiel in Form eines Hall-Sensors) detektiert die Veränderung der Magnetfeldstärke eines Magnetfelds im Bereich der Aufnahme, die durch das Einsetzen des Verbindungsstifts in die Aufnahme bewirkt wird.
Selbstverständlich kann die Signalabgabe auch durch andere bekannte Detektionsverfahren ausgelöst werden, wie zum Beispiel durch induktive oder kapazitive Erkennung des Verbindungsstifts, durch Herstellung eines elektrischen Kontakts durch das Einsetzen des Verbindungsstifts in die Aufnahme oder ein bilderkennendes Verfahren mittels Bildwandler (CCD oder CMOS-Baustein).

Wird als Sensor ein Drehmomentsensor verwendet, so besteht in einem besonders bevorzugten Ausführungsbeispiel die Möglichkeit, diesen Drehmomentsensor sowohl zum Nachweis der erfolgten Verbindung der Drehmomentbremse mit der Werkzeughalterung zu verwenden, als auch als Drehmoment-Meßvorrichtung während des Kalibrierverfahrens. Hierzu wird das erste vom Drehmomentsensor kommende Signal von der Auswerte-Elektronik lediglich als Startsignal zum Start des Verfahrens zur Bestimmung des Motorstroms verarbeitet, wo hingegen die darauf folgenden Signale als Teil des Kalibrierverfahrens ausgewertet werden.

In einem zweiten Ausführungsbeispiel ist das Element zur Signalabgabe als Schaltelement ausgebildet, bestehend aus einem Schaltkontakt und einem Schaltglied, das durch das Einstecken des Verbindungsstifts in die Aufnahme ein Schaltsignal auslöst. Die Verarbeitung des Schaltsignals erfolgt in gleicher Weise wie für ein Signal eines Sensors.

Das Schaltelement kann in oder an der Aufnahme angeordnet sein, wobei zumindest das Schaltglied in die Aufnahme hinein reicht. Durch Einstecken des Verbindungsstifts in die Aufnahme wird das Schaltglied aus seiner Ruhestellung bewegt, die Spannungszuführung über den Schaltkontakt geschlossen und ein Schaltsignal ausgelöst.

Als Drehmomentbremse der Vorrichtung zur Bestimmung des Motorstroms können bevorzugt Hysteresebremsen, mechanische Seilbremsen, Trommelbremsen, Magnetbremsen, Fluidbremsen oder auf elektrischer oder mechanischer Reibung basierende Bremsen verwendet werden.

Gemäß Anspruch 12 kann die erfindungsgemäße Vorrichtung zur Bestimmung des zum Aufbringen eines Drehmoments benötigten Werts des Motorstroms in eine Steuervorrichtung für das Handstück und dessen Antrieb, bevorzugt in der Form eines Tischgeräts, integriert sein, wodurch eine kompakte und für den Anwender einfach zu bedienende Kompletteinheit entsteht.

Das erfindungsgemäße Verfahren nach Anspruch 13 mit den Schritten
a) Abgabe eines Signals durch ein Element zur Signalabgabe, bevorzugt ein Sensor oder ein Schaltelement, bevorzugt aufgrund der Detektion der Veränderung der Drehzahl, des Drehmoments, des Drucks, der Lichtstärke, der Lautstärke, der Magnetfeldstärke, der Induktion oder der Kapazität,
b) Empfang und Verarbeitung des Signals und
c) Start der Bestimmung des zum Aufbringen eines Drehmoments benötigten Werts des Motorstroms vor der Benutzung des Handstücks durch die Auswerte-Elektronik
erlaubt es dem Anwender das Kalibrierverfahren ohne Berühren des Bedienfelds bzw. der Tasten durchzuführen. Die Auswerte-Elektronik kann als Teil eines Mikroprozessors ausgeführt sein, der den gesamten Ablauf des Verfahrens steuert.

In einem Ausführungsbeispiel wählt der Anwender in einem ersten Schritt ein Betriebsprogramm oder einzelne Betriebsparameter, insbesondere das Drehmoment, die Drehzahl oder das Übersetzungsverhältnis, für den Betrieb des Handstücks und abgestimmt auf die bevor stehende Behandlung über das Bedienfeld der Steuervorrichtung aus. Dabei kann der Anwender entweder selbst Werte eingeben oder aus voreingestellten Betriebsprogrammen ein gewünschtes Programm auswählen. Anschließend steckt er den Verbindungsstift, der in die Werkzeughalterung des Handstücks eingespannt ist, in die Aufnahme der Drehmomentbremse. Dadurch bzw. durch die Betätigung einer Fuß- oder Fingersteuerung oder einer Fernbedienung, die mit der Steuervorrichtung, dem Motor oder dem Handstück in Wirkverbindung steht, kommt es zur Änderung eines Parameters, bevorzugt der Drehzahl, des Drehmoments, des Drucks, der Lichtstärke einer Strahlungsquelle, der Lautstärke eines akustischen Signals, der Magnetfeldstärke, der Induktion oder der Kapazität. Die Änderung wird vom Sensor erfaßt bzw. bewirkt das Schalten des Schaltelements und in der Folge die Abgabe eines Signals gemäß Schritt a).

In einem bevorzugten Ausführungsbeispiel wird der vom Anwender ausgewählte Wert der Drehzahl oder des Drehmoments oder das ausgewählte Übersetzungsverhältnis mit einem gespeicherten Sollwert oder Soll-Bereich verglichen und wenn der Sollwert / Soll-Bereich nicht eingehalten wird, das Verfahren vor dem Schritt c) abgebrochen. Dadurch wird erreicht, daß der Anwender nur bis zu einem vorbestimmten Maximal- bzw. Minimalwert oder innerhalb eines vorgegebenen Bereichs das Kalibrierverfahren durchführen kann, was insbesondere die Sicherheit für die darauf folgende Behandlung erhöht.

Die Erfindung wird nachfolgend anhand bevorzugter Ausführungsbeispiels und Bezug nehmend auf die beigefügten Zeichnungen erläutert:
- Figur 1: zeigt eine schematische Darstellung der erfindungsgemäßen Vorrichtung zur Bestimmung des Motorstroms mit den Signalflüssen.
- Figur 2: zeigt die der erfindungsgemäßen Vorrichtung zur Bestimmung des Motorstroms mit einem Drucksensor.
- Figur 3: der erfindungsgemäßen Vorrichtung zur Bestimmung des Motorstroms mit einem Drehzahlsensor.
- Figur 4: der erfindungsgemäßen Vorrichtung zur Bestimmung des Motorstroms mit einem Lichtsensor.
- Figur 5: der erfindungsgemäßen Vorrichtung zur Bestimmung des Motorstroms mit einem Drehmomentsensor.
- Figur 6: der erfindungsgemäßen Vorrichtung zur Bestimmung des Motorstroms mit einem Schalter.

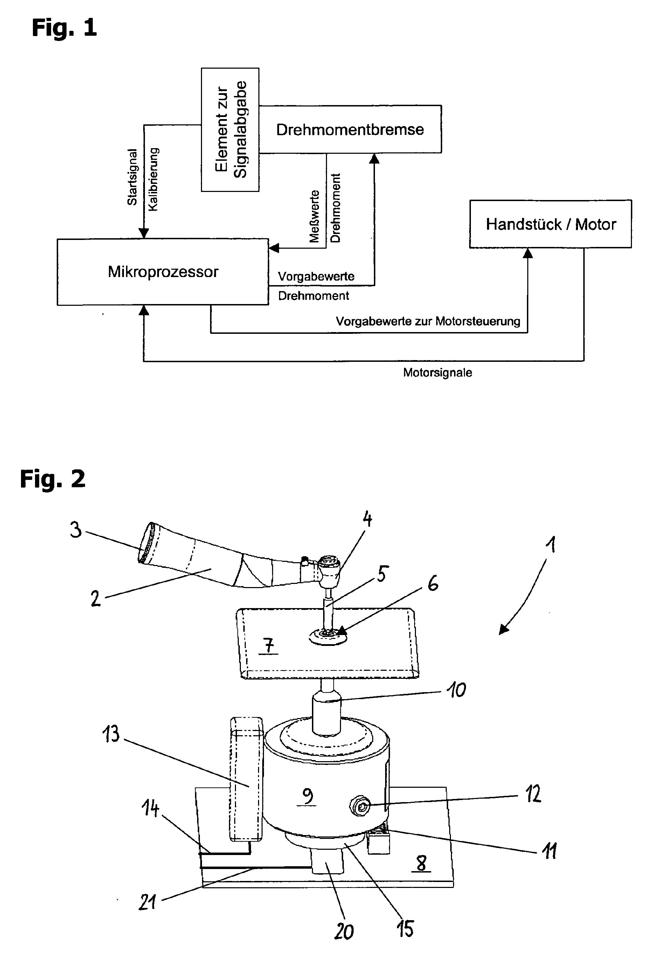

Wie aus Figur 1 zu erkennen ist, sendet das Element zur Signalabgabe der erfindungsgemäßen Vorrichtung zur Bestimmung des Motorstroms ein Signal an den Mikroprozessor zum Start des Kalibrierverfahrens. Die Signalabgabe erfolgt, nachdem das Element zur Signalabgabe die Verbindung der Drehmomentbremse mit der Werkzeughalterung des Handstücks aufgrund von Veränderung eines Parameters, bevorzugt der Drehzahl, des Drehmoments, des Drucks, der Lichtstärke, der Lautstärke, der Magnetfeldstärke, der Induktion oder der Kapazität, detektiert hat. Die Auswerte-Elektronik des Mikroprozessors verarbeitet das Signal und ruft aus einem Speicher das Kalibrierverfahren auf. Zum Start des Kalibriervefahrens wird/werden der Drehmomentbremse ein oder mehrere Bremswerte vorgegeben, abhängig davon ob die Kalibrierung nur für einen oder mehrere Werte erfolgen soll. Der maximale Wert der Bremswert-Vorgabe entspricht jenem maximalen Drehmoment, das der Anwender vorher eingegeben hat bzw. über die Auswahl eines Programms bestimmt hat. Gleichzeitig wird der Motor des Handstücks gestartet und er erhält alle zur Motorsteuerung benötigten Daten bzw. sendet die dafür benötigten Signale, z.B. von Hall-Sensoren zur Kommutierung, an die Steuerelektronik, die ebenfalls als Teil des Mikroprozessors ausgeführt ist. Eine Motorstrom-Meßvorrichtung als Teil des Mikroprozessors mißt den zum Aufbringen des Drehmoments benötigten Wert des Motorstroms. Diese Werte sowie die zugehörigen Drehmoment-Meßwerte, die von der Drehmomentbremse oder einer ihr zugeordneten Drehmoment-Meßvorrichtung an den Mikroprozessor weitergeleitet werden, werden in einem Speicher abgelegt. Bei der an den Kalibriervorgang anschließenden Anwendung des Handstücks, zum Beispiel einem chirurgischen Eingriff, insbesondere dem Setzen eines Zahnimplantats, wird der gespeicherte Wert des Motorstroms wieder aufgerufen, wodurch garantiert ist, daß exakt das gewünschte Drehmoment aufgebracht wird.

Die Figuren 2 - 6 zeigen jeweils die erfindungsgemäße Vorrichtung zur Bestimmung des zum Aufbringen eines Drehmoments benötigten Werts des Motorstroms für medizinische Handstücke mit unterschiedlichen Elementen zur Signalabgabe. Gleiche Bauteile sind in allen Darstellungen mit den selben Ziffern beschriftet.

In allen Figuren 2 - 6 ist die Vorrichtung 1 mit einem Handstück 2 dargestellt. Der Motor zum Antrieb des Werkzeugs ist entweder in das Handstück 2 integriert oder er ist über eine Kupplungsvorrichtung 3 an das Handstück 2 angeschlossen. Im Kopf 4 der Handstücks 2 ist eine Aufnahme (Spannvorrichtung) angeordnet, in die das für die Behandlung benötigte Werkzeug eingespannt ist. Zur Durchführung des Kalibrierverfahrens wird jedoch bevorzugt an Stelle des Werkzeugs ein spezieller Verbindungsstift 5, der für die Durchführung des Verfahrens besser zu handhaben ist, verwendet. Das gegenüber liegende Ende des Verbindungsstifts ist in die Aufnahmeöffnung 6 der erfindungsgemäßen Vorrichtung 1 eingesetzt. Der Kalibriervorgang könnte jedoch auch mit einem eingespannten Werkzeug (Bohrer) erfolgen.

Die erfindungsgemäße Vorrichtung 1 ist in einem Gehäuse angeordnet, dessen Wände 7, 8 ausschnittsweise dargestellt sind. Ist die Vorrichtung 1 als Teil der Steuervorrichtung für das Handstück und dessen Antrieb ausgebildet, so sind die Wände 7, 8 Teile des Gehäuses der Steuervorrichtung.

Die erfindungsgemäße Vorrichtung 1 besteht aus einer Drehmomentbremse 9 (Hysteresebremse), die über eine Aufnahme 10 lösbar mit dem Verbindungsstift 5 verbunden ist und in einer Lagervorrichtung 15 gelagert ist. Die Federkraft der Feder 11 dient als Gegenkraft zur Verdrehung der Drehmomentbremse 9 während des Kalibrierverfahrens sowie zur Rückstellung der Drehmomentbremse 9 in ihr Ausgangsstellung nach Beendigung des Kalibrierverfahrens. Über die Öffnung 12 erfolgt die Stromzufuhr für die Hysteresebremse 9, die durch die Elektronik des Mikroprozessors entsprechend dem ausgewählten Drehmomentwert festgelegt wird. Mit 13 ist eine Drehmoment-Meßvorrichtung bezeichnet, die während des Kalibrierverfahrens das durch den Verbindungsstift 5 übertragene Drehmoment mißt. Die ermittelten Werte werden über die Leiterbahn 14 einer Auswerte-Elektronik zugeführt. Die Drehmoment-Meßvorrichtung 13 wird insbesondere bei Drehmomentbremsen, bei denen die Bremskraft unbekannt bzw. nicht exakt einstellbar ist (z.B. mechanische Seilbremsen, Trommelbremsen oder auf elektrischer oder mechanischer Reibung basierende Bremsen), benötigt.

Im folgenden wird für jede Abbildung einzeln das dargestellte Element zur Signalabgabe und dessen Funktion erläutert.

In Figur 2 ist unterhalb der Drehmomentbremse 9 ein Drucksensor 20, z.B. in Form eines Piezoelements oder eines FSR-Drucksensors, angeordnet. Durch einen leichten Druck, den der Anwender nach dem Einstecken des Verbindungsstifts 5 in die Aufnahmeöffnung 6 ausübt, werden die Aufnahme 10 und die Drehmomentbremse 9 gegen die Kraft der Lagervorrichtung 15 nach unten (in Richtung Gehäusewand 8) gedrückt. Der Drucksensor 20 detektiert die auf ihn ausgeübte Kraft und sendet ein Signal aus, das über die Leitung 21 der Auswerte-Elektronik übermittelt wird und den Kalibriervorgang auslöst. Beendet der Anwender die Druckausübung, so werden die Drehmomentbremse 9 und die Aufnahme 10 durch die Kraft der Lagervorrichtung 15 wieder in ihre Ausgangsposition zurück bewegt.

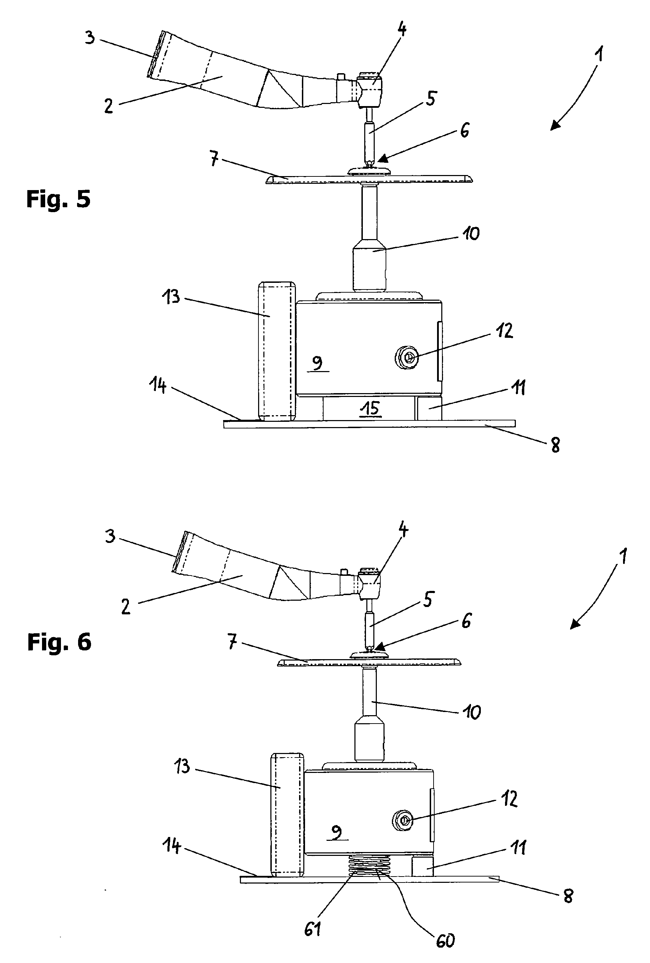

In Figur 6 ist an Stelle des Drucksensors ein selbst rückstellender Schalter 60 in unmittelbarer Nähe der Drehmomentbremse 9 angeordnet. Durch einen leichten Druck, den der Anwender nach dem Einstecken des Verbindungsstifts 5 in die Aufnahmeöffnung 6 ausübt, werden die Aufnahme 10 und die Drehmomentbremse 9 gegen die Kraft der Feder 61 nach unten (in Richtung Gehäusewand 8) verschoben. Die Feder 61 dient auch der Rückbewegung der Drehmomentbremse 9 und der Aufnahme 10 in ihre Ausgangsposition nach Abschluß der Druckausübung durch den Anwender. Die nach unten (in Richtung der Gehäusewand 8) gedrückte Drehmomentbremse 9 löst in bekannter Weise das Schaltglied aus, wodurch der Schaltkontakt geschlossen wird und ein Schaltsignal ausgelöst wird.

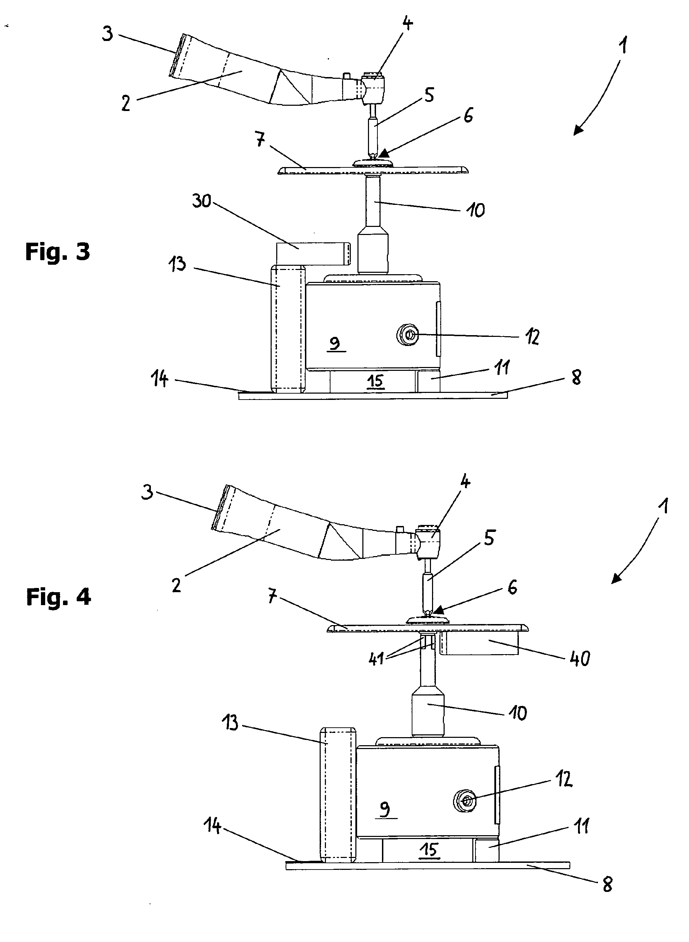

In Figur 3 wird der Start des Kalibrierverfahrens durch ein Signal eines stationären Drehzahlsensor 30 in Form einer Hell-Dunkel-Photozelle ausgelöst. Der Drehzahlsensor 30 ist in unmittelbarer Nähe zur Aufnahme 10 angeordnet und detektiert die Rotation der Aufnahme 10, insbesondere die Rotationsbewegung einer auf der Aufnahme 10 angebrachten Markierung. Die Rotationsbewegung wird vom Motor des Handstücks über den Verbindungsstift 5 auf die Aufnahme 10 übertragen, wobei der Motor durch die Betätigung einer Fuß- oder Fingersteuerung oder einer Fernbedienung gestartet wird.

Der Einsatz eines Drehzahlsensors 30 ist besonders vorteilhaft, da während des Kalibriervorgangs zusätzlich noch das Übersetzungsverhältnis (Motordrehzahl : Bohrerdrehzahl) des Handstücks 2 bestimmt werden kann. Die Motordrehzahl wird als Grundlage für die Ansteuerung des Motors während des Betriebs ständig bestimmt, die Bohrerdrehzahl entspricht der Drehzahl der Aufnahme 10. Aus deren Verhältnis kann mittels entsprechender Elektronik das aktuelle Übersetzungsverhältnis bestimmt werden. Das aktuelle Übersetzungsverhältnis kann in einem weiteren Schritt nun mit dem vom Anwender über das Bedienfeld der Steuervorrichtung ausgewählten Übersetzungsverhältnis verglichen werden, wobei jedes Übersetzungsverhältnis einem bestimmten Handstück zugeordnet ist. Stimmen beide Übersetzungsverhältnisse überein, so kann das Kalibrierverfahren fortgesetzt werden, stimmen die beiden Übersetzungsverhältnisse nicht überein, so hat der Anwender entweder ein falsches Handstück in Verwendung bzw. über die Steuervorrichtung ein falsches Übersetzungsverhältnis (Handstück) in Bezug auf das verwendete Handstück ausgewählt. In diesem Fall wird das Kalibrierverfahren aus Sicherheitsgründen automatisch abgebrochen oder automatisch das passende Übersetzungsverhältnis eingestellt.

Auch der in Figur 4 dargestellte Sensor 40 umfaßt eine Photozelle, die in diesem Fall jedoch die Lichtstärke der Strahlung einer Lichtquelle detektiert, die durch das Einsetzen das Verbindungsstifts 5 in die Aufnahme 10 verändert wird. Die Lichtquelle, z.B. eine Infrarot-Lichtquelle in Form einer LED, ist Teil des Sensors 40 oder als eigenes Bauteil neben dem Sensor 40 angeordnet. Der Sensor 40 ist direkt unterhalb der Gehäusewand 7 im obersten Abschnitt der Aufnahme 10, wo auch die Spitze des Verbindungsstifts 5 noch hinreicht, angeordnet. Im Bereich des Sensors 40 sind in die Aufnahme mehrere schlitzförmige Durchgangsbohrungen 41 eingefräst, durch die das Licht der Lichtquelle in das Innere der Aufnahme 10 fällt. Aufgrund der dunklen Oberfläche der Aufnahme sowie des Inneren der Aufnahme wird nur wenig Licht reflektiert. Wird der Verbindungsstift 5 in die Aufnahme 10 gesteckt und ist das Vorderende des Verbindungsstifts 5 mit einer hellen, stark reflektierenden Oberfläche versehen, so wird dadurch mehr Licht reflektiert. Der Sensor 40 detektiert die erhöhte Lichtstärke und gibt ein Signal zum Start des Kalibrierverfahrens ab.

Alternativ kann die Lichtquelle gegenüber dem Sensor 40 angeordnet sein, so daß Licht durch eine oder mehrere Durchgangsbohrungen 41 auf der Sensor 40 fällt und eine Lichtschranke entsteht. Setzt der Anwender den Verbindungsstift 5 in die Aufnahme 10, so wird die Lichtschranke unterbrochen bzw. die Lichtstärke verringert. Der Sensor 40 detektiert diese Veränderung und gibt ein Signal zum Start des Kalibrierverfahrens ab.

In Figur 5 wird als Sensor zum Start des Kalibrierverfahrens der Drehmomentsensor 13 verwendet, der während des Kalibrierverfahrens das Drehmoment mißt. Der Anwender setzt den Verbindungsstift 5 in die Aufnahme 10, betätigt die Fuß- oder Fingersteuerung oder eine Fernbedienung zum Start des Motors, wodurch der Verbindungsstift 5 und die Aufnahme 10 in Rotation versetzt werden. Die Drehmomentbremse 9, deren Bremsvorrichtung schon aktiviert /vorgespannt ist (wie auch bei der Verwendung eines Sensors der die Drehzahl mißt, siehe Figur 3), wird ebenfalls verdreht und der Drehmomentsensor 13 detektiert das übertragene Moment. Das erste vom Drehmomentsensor 13 kommende Signal wird von der Auswerte-Elektronik als Startsignal zum Start des Verfahrens zur Bestimmung des Motorstroms verarbeitet, die darauf folgenden Signale als Teil des Kalibrierverfahrens ausgewertet. Durch diesen Aufbau entfällt die Notwendigkeit zusätzliche Sensoren oder Schalter in die Vorrichtung 1 einzubauen.

Die Erfindung ist nicht auf die beschriebenen Ausführungsbeispiele beschränkt, sondern umfaßt alle Ausführungsmöglichkeiten, die das prinzipielle, sinngemäßen Funktionsprinzip der Erfindung nicht verändern.

## Patentansprüche

1. Vorrichtung (1) zur Bestimmung des zum Aufbringen eines Drehmoments benötigten Werts des Motorstroms für medizinische Handstücke mit einer Drehmomentbremse (9), die mit der Werkzeughalterung des Handstücks (2) verbindbar ist, und einer Motorstrom-Meßvorrichtung für den Handstückmotor, **gekennzeichnet durch**
ein Element zur Signalabgabe (13, 20, 30, 40, 60), bevorzugt ein Sensor oder ein Schaltelement, das ein Signal zum Start eines Verfahrens zur Bestimmung des zum Aufbringen eines Drehmoments benötigten Werts des Motorstroms für medizinische Handstücke abgibt.

2. Vorrichtung nach Anspruch 1, **dadurch gekennzeichnet, daß**
die Vorrichtung (1) zusätzlich eine Drehmoment-Meßvorrichtung (13) aufweist.

3. Vorrichtung nach Anspruch 1 oder 2, **gekennzeichnet durch**
einen Auswerte- und Steuerelektronik, bevorzugt als Teil eines Mikroprozessors, die das Signal des Elements zur Signalabgabe empfängt, verarbeitet und das Verfahren zur Bestimmung des zum Aufbringen eines Drehmoments benötigten Werts des Motorstroms startet.

4. Vorrichtung nach einem der vorhergehenden Ansprüche, **gekennzeichnet durch**
einen Speicher, in dem das Verfahren zur Bestimmung des Motorstroms als Programm abgelegt ist.

5. Vorrichtung nach einem der vorhergehenden Ansprüche, **dadurch gekennzeichnet, daß**
die Verbindung zwischen der Drehmomentbremse (9) und der Werkzeughalterung des Handstücks (2) mittels eines Verbindungsstifts (5) erfolgt, daß die Drehmomentbremse (9) mit einer Aufnahme (6, 10) für einen Verbindungsstift (5) versehen ist und daß der Sensor (13, 20, 30, 40) oder Teile des Sensors an die Aufnahme (6, 10) angrenzen oder in diese ragen.

6. Vorrichtung nach einem der vorhergehenden Ansprüche, **dadurch gekennzeichnet, daß**
der Sensor (13, 20, 30, 40) eine Veränderung der Drehzahl, des Drehmoments, des Drucks, der Lichtstärke, der Lautstärke, der Magnetfeldstärke, der Induktion oder der Kapazität detektiert oder ein Bildwandler ist.

7. Vorrichtung nach Anspruch 1 oder 2, **dadurch gekennzeichnet, daß**
der Sensor ein Drehmomentsensor (13) ist und daß der Drehmomentsensor (13) auch der Messung des Drehmoments während des Kalibrierverfahrens dient.

8. Vorrichtung nach Anspruch 1, **dadurch gekennzeichnet, daß**
der Sensor ein Drehzahlsensor (30), bevorzugt eine Photozelle, ist, dessen Meßwerte zusätzlich zur Bestimmung des Übersetzungsverhältnisses (Motordrehzahl : Bohrerdrehzahl) verwendet werden.

9. Vorrichtung nach einem der Ansprüche 1 - 4, **dadurch gekennzeichnet, daß**
das Schaltelement ein selbst rückstellender Schalter (60) ist, der in oder an der Aufnahme (10) angeordnet ist und durch das Einstecken des Verbindungsstifts (5) in die Aufnahme (10) betätigt wird.

10. Vorrichtung nach einem der Ansprüche 1 - 4, **dadurch gekennzeichnet, daß**
das Schaltelement ein selbst rückstellender Schalter (60) ist, der in unmittelbarer Nähe der Drehmomentbremse (9) angeordnet ist und daß durch eine Relativbewegung der Drehmomentbremse (9) in Bezug auf den Schalter (60) der Schalter (60) betätigt und der Schaltkontakt geschlossen wird.

11. Vorrichtung nach einem der vorhergehenden Ansprüche, **dadurch gekennzeichnet, daß**
die Drehmomentbremse (9) eine Hysteresebremse, mechanische Seilbremse, Trommelbremse, Magnetbremse, Fluidbremse oder eine auf elektrischer oder mechanischer Reibung basierende Bremse ist.

12. Steuervorrichtung zum Antrieb eines medizinischen Handstücks, **gekennzeichnet durch**
eine Vorrichtung (1) zur Bestimmung des Motorstroms nach einem der Ansprüche 1 - 11.

13. Verfahren zur Bestimmung des Motorstroms, **gekennzeichnet durch** die Schritte
a) Abgabe eines Signals **durch** ein Element zur Signalabgabe (13, 20, 30, 40, 60), bevorzugt ein Sensor oder ein Schaltelement,
b) Empfang und Verarbeitung des Signals und
c) Start des Verfahrens zur Bestimmung des zum Aufbringen eines Drehmoments benötigten Werts des Motorstroms vor der Benutzung des Handstücks (2) **durch** die Auswerte-Elektronik.

14. Verfahren nach Anspruch 13, **dadurch gekennzeichnet, daß**
vor dem Schritt a) in einem Schritt a1) ein Verbindungsstift (5), der in die Werkzeughalterung des Handstücks eingespannt ist, in eine Aufnahme (6, 10) der Drehmomentbremse (9) gesteckt wird.

15. Verfahren nach Anspruch 13 oder 14, **dadurch gekennzeichnet, daß**
vor dem Schritt a) oder a1) der Anwender ein Betriebsprogramm oder einzelne Betriebsparameter, insbesondere die Drehzahl, das Drehmoment oder das Übersetzungsverhältnis für den Betrieb des Handstücks (2) über das Bedienfeld der Steuervorrichtung auswählt.

16. Verfahren nach Anspruch 15, **dadurch gekennzeichnet, daß**
der vom Anwender ausgewählte Wert der Drehzahl, des Drehmoments oder des Übersetzungsverhältnisses mit einem gespeicherten Sollwert oder Soll-Bereich verglichen wird und wenn der Sollwert / Soll-Bereich nicht eingehalten wird, das Verfahren vor dem Schritt c) abgebrochen wird.

17. Verfahren nach einem der Ansprüche 13 bis 16, **dadurch gekennzeichnet, daß**
eine Veränderung der Drehzahl oder des Drehmoments die Abgabe eines Signals gemäß Schritt a) auslöst und daß die Veränderung der Drehzahl oder des Drehmoments durch die Betätigung einer Fuß- oder Fingersteuerung oder einer Fernbedienung, die mit der Steuervorrichtung, dem Motor oder dem Handstück (2) in Wirkverbindung steht, ausgelöst wird.
